(19) Europäisches Patentamt
European Patent Office
Office européen des brevets

(11) Veröffentlichungsnummer: **0 444 286 A1**

# (12) EUROPÄISCHE PATENTANMELDUNG

(21) Anmeldenummer: **90124680.1**

(22) Anmeldetag: **19.12.90**

(51) Int. Cl.5: **C07D 239/28**, C07D 239/34, C07D 401/12, C07D 403/12, C07D 409/12, C07D 417/12, A01N 43/54

(30) Priorität: **01.03.90 DE 4006394**
**13.09.90 DE 4029055**

(43) Veröffentlichungstag der Anmeldung:
**04.09.91 Patentblatt 91/36**

(84) Benannte Vertragsstaaten:
**BE CH DE DK ES FR GB IT LI NL**

(71) Anmelder: **BAYER AG**

**W-5090 Leverkusen 1 Bayerwerk(DE)**

(72) Erfinder: **Kirsten, Rolf, Dr.**
**Carl-Langhans-Strasse 27**
**W-4019 Monheim(DE)**
Erfinder: **Wolf, Hilmar, Dr.**
**Zum Brauhaus 14**
**W-4018 Langenfeld(DE)**
Erfinder: **Lürssen, Klaus, Dr.**
**August-Kierspel-Strasse 145**
**W-5060 Bergisch Gladbach(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

## (54) Sulfonylierte Pyrimidincarbonsäureamide.

(57) Die Erfindung betrifft neue sulfonylierte Pyrimidincarbonsäureamide der Formel (I),

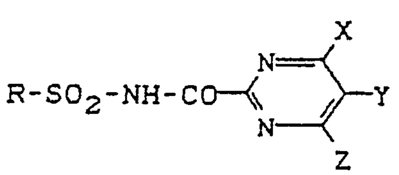

(I)

in welcher
R für jeweils gegebenenfalls substituiertes Aryl, Aralkyl oder Heteroaryl steht,
X für Wasserstoff, Halogen, Alkyl, Halogenalkyl, Alkoxyalkyl, Alkoxy oder Halogenalkoxy steht,
Y für Halogen, Alkyl, Halogenalkyl, Alkoxyalkyl, Alkoxy oder Halogenalkoxy steht und
Z für Wasserstoff, Halogen, Alkyl, Halogenalkyl, Alkoxy oder Halogenalkoxy steht,
sowie Salze von Verbindungen der Formel (I), ferner Verfahren zu deren Herstellung und deren Verwendung als Herbizide.

EP 0 444 286 A1

Die Erfindung betrifft neue sulfonylierte Pyrimidincarbonsäureamide, Verfahren zu ihrer Herstellung und ihre Verwendung als Herbizide.

Es ist bereits bekannt, daß bestimmte heterocyclische N-Acylsulfonamide, wie z.B. N-(2-Methoxy-phenylsulfonyl)-4,6-dimethyl-pyrimidin-2-carbonsäureamid und N-(2-Chlor-phenylsulfonyl)-4,6-dimethoxy-pyrimidin-2-carbonsäureamid, herbizide Eigenschaften aufweisen (vgl. EP-A 353640).

Die herbizide Wirksamkeit dieser Verbindungen ist jedoch nicht immer ganz zufriedenstellend.

Es wurden nun die neuen sulfonylierten Pyrimidincarbonsäureamide der allgemeinen Formel (I)

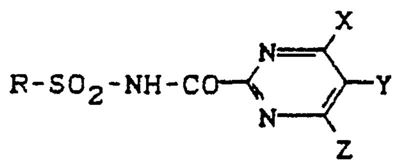

(I)

in welcher

R für jeweils gegebenenfalls substituiertes Aryl, Aralkyl oder Heteroaryl steht,

X für Wasserstoff, Halogen, Alkyl, Halogenalkyl, Alkoxyalkyl, Alkoxy oder Halogenalkoxy steht,

Y für Halogen, Alkyl, Halogenalkyl, Alkoxyalkyl, Alkoxy oder Halogenalkoxy steht und

Z für Wasserstoff, Halogen, Alkyl, Halogenalkyl, Alkoxy oder Halogenalkoxy steht,

sowie Salze von Verbindungen der Formel (I) gefunden.

Man erhält die neuen sulfonylierten Pyrimidincarbonsäureamide der allgemeinen Formel (I), wenn man

(a) Pyrimidincarbonsäureamide der allgemeinen Formel (II)

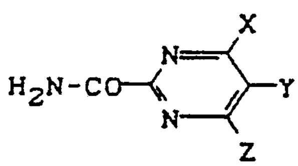

(II)

in welcher

X, Y und Z die oben angegebenen Bedeutungen haben,

mit Sulfonsäurehalogeniden oder Sulfonsäureanhydriden der allgemeinen Formel (III)

$R-SO_2-Q$ (III)

in welcher

R die oben angegebene Bedeutung hat und

Q für Fluor, Chlor, Brom oder die Gruppierung $-O-SO_2-R$ steht, worin R die oben angegebene Bedeutung hat,

gegebenenfalls in Gegenwart eines Säureakzeptors und gegebenenfalls in Gegenwart eines Verdünnungsmittels umsetzt, oder wenn man

(b) Pyrimidincarbonsäuren der allgemeinen Formel (IV)

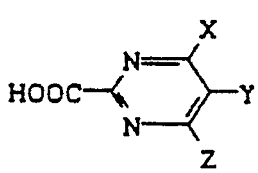

(IV)

in welcher

X, Y und Z die oben angegebenen Bedeutungen haben,

oder reaktionsfähige Derivate von Verbindungen der Formel (IV)

mit Sulfonsäureamiden der allgemeinen Formel (V)

$R-SO_2-NH_2$ (V)

in welcher

R die oben angegebene Bedeutung hat,

oder mit reaktionsfähigen Derivaten von Verbindungen der Formel (V)

gegebenenfalls in Gegenwart von Reaktionshilfsmitteln und gegebenenfalls in Gegenwart von Verdünnungsmitteln umsetzt

und gegebenenfalls die nach den Verfahren (a) oder (b) erhaltenen Produkte nach üblichen Methoden in Salze überführt.

Die neuen sulfonylierten Pyrimidincarbonsäureamide der allgemeinen Formel (I) zeichnen sich durch starke herbizide Wirksamkeit aus.

Überraschenderweise zeigen die erfindungsgemäßen sulfonylierten Pyrimidincarbonsäureamide der Formel (I) erheblich stärkere herbizide Wirkung als die aus dem Stand der Technik bekannten heterocyclischen N-Acylsulfonamide, wie z.B. N-(2-Methoxy-phenylsulfonyl)-4,6-dimethylpyrimidin-2-carbonsäureamid und N-(2-Chlor-phenylsulfonyl)-4,6-dimethoxy-pyrimidin-2-carbonsäureamid.

Gegenstand der Erfindung sind vorzugsweise Verbindungen der Formel (I), in welcher

R für den Rest

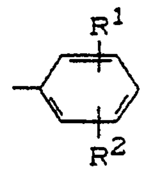

steht, worin

$R^1$ und $R^2$

gleich oder verschieden sind und für Wasserstoff, Fluor, Chlor, Brom, Iod, Cyano, Nitro, Carboxy, $C_1$-$C_6$-Alkyl (welches gegebenenfalls durch Fluor, Chlor, Brom, Cyano, Carboxy, $C_1$-$C_4$-Alkoxycarbonyl, $C_1$-$C_4$-Alkylamino-carbonyl, Di-($C_1$-$C_4$-alkyl)-amino-carbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist), für $C_2$-$C_6$-Alkenyl (welches gegebenenfalls durch Fluor, Chlor, Brom, Cyano, $C_1$-$C_4$-Alkoxycarbonyl, Carboxy oder Phenyl substituiert ist), für $C_2$-$C_6$-Alkinyl (welches gegebenenfalls durch Fluor, Chlor, Brom, Cyano, $C_1$-$C_4$-Alkoxy-carbonyl, Carboxy oder Phenyl substituiert ist), für $C_1$-$C_4$-Alkoxy (welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist), für $C_1$-$C_4$-Alkylthio (welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist), für $C_3$-$C_6$-Alkenyloxy (welches gegebenenfalls durch Fluor, Chlor, Brom, Cyano oder $C_1$-$C_4$-Alkoxy-carbonyl substituiert ist), für $C_2$-$C_6$-Alkenylthio (welches gegebenenfalls durch Fluor, Chlor, Brom, Cyano, Nitro, $C_1$-$C_3$-Alkylthio oder $C_1$-$C_4$-Alkoxycarbonyl substituiert ist), $C_3$-$C_6$-Alkinyloxy, $C_3$-$C_6$-Alkinylthio oder für den Rest $-S(O)_p-R^3$ stehen, wobei

p

für die Zahlen 1 oder 2 steht und

$R^3$

für $C_1$-$C_4$-Alkyl (welches gegebenenfalls durch Fluor, Chlor, Brom, Cyano oder $C_1$-$C_4$-Alkoxy-carbonyl substituiert ist), $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino oder für den Rest $-NHOR^4$ steht, wobei

$R^4$

für $C_1$-$C_6$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist), für $C_3$-$C_6$-Alkenyl (welches gegebenenfalls durch Fluor, Chlor oder Brom substituiert ist), $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist), für Benzhydryl oder für Phenyl (welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-

Alkoxy-carbonyl substituiert ist) steht,

$R^1$ und $R^2$

weiterhin für Phenyl oder Phenoxy, für $C_1$-$C_4$-Alkylcarbonylamino, $C_1$-$C_4$-Alkoxycarbonylamino, $C_1$-$C_4$-Alkylamino-carbonylamino, Di-($C_1$-$C_4$-alkyl)-amino-carbonylamino, oder für den Rest -CO-$R^5$ stehen, wobei

$R^5$

für $C_1$-$C_6$-Alkyl, $C_1$-$C_6$-Alkoxy (welches gegebenenfalls durch Fluor, Chlor, Methoxy oder Ethoxy substituiert ist), $C_3$-$C_6$-Cycloalkoxy, $C_3$-$C_6$-Alkenyloxy, $C_1$-$C_4$-Alkylthio, Amino, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht (welche gegebenenfalls durch Fluor und/oder Chlor substituiert sind),

$R^1$ und $R^2$

weiterhin für $C_1$-$C_4$-Alkylsulfonyloxy, Di-($C_1$-$C_4$-alkyl)-aminosulfonylamino oder für den Rest -CH=N-$R^6$ stehen, wobei

$R^6$

für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/ oder Chlor substituiertes $C_1$-$C_6$-Alkoxy, $C_3$-$C_6$-Alkenoxy, $C_3$-$C_6$-Alkinoxy oder Benzyloxy, für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkyl-sulfonylamino oder für gegebenenfalls durch Fluor, Chlor, Brom oder Methyl substituiertes Phenylsulfonylamino steht,

worin weiter

R für den Rest

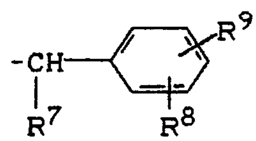

steht, worin

$R^7$

für Wasserstoff oder $C_1$-$C_4$-Alkyl steht,

$R^8$ und $R^9$

gleich oder verschieden sind und für Wasserstoff, Fluor, Chlor, Brom, Nitro, Cyano, Carboxy, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), $C_1$-$C_4$-Alkoxy-carbonyl, $C_1$-$C_4$-Alkylsulfonyl oder Di-($C_1$-$C_4$-alkyl)-aminosulfonyl stehen; worin weiter

R für den Rest

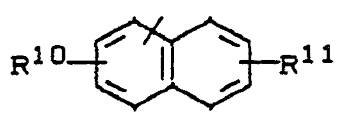

steht, worin

$R^{10}$ und $R^{11}$

gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist) oder $C_1$-$C_4$-Alkoxy (welches gegebenenfalls durch Fluor und/oder Chlor substituiert ist), stehen; worin weiter

R für den Rest

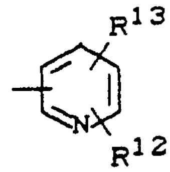

steht, worin

$R^{12}$ und $R^{13}$

gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_2$-$C_4$-Alkenyl (welches gegebenenfalls durch Fluor und/oder Chlor substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), sowie für Di-($C_1$-$C_4$-alkyl)-aminosulfonyl, $C_1$-$C_4$-Alkoxy-carbonyl, Dimethylaminocarbonyl oder Dioxolanyl stehen; worin weiter

R für den Rest

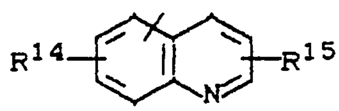

steht, worin

$R^{14}$ und $R^{15}$

gleich oder verschieden sind und für Wasserstoff, Fluor, Chlor, Brom, $C_1$-$C_4$-Alkyl (welches gegebenenfalls durch Fluor und/oder Brom substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), oder für Di-($C_1$-$C_4$-alkyl)-aminosulfonyl stehen; worin weiter

R für den Rest

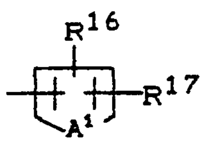

steht, worin

$R^{16}$ und $R^{17}$

gleich oder verschieden sind und für Wasserstoff, Fluor, Chlor, Brom, Cyano, Nitro, $C_1$-$C_4$-Alkyl (welches gegebenenfalls durch Fluor, Chlor, $C_1$-$C_4$-Alkoxy und/oder $C_1$-$C_4$-Halogenalkoxy substituiert ist), $C_1$-$C_4$-Alkoxy (welches gegebenenfalls durch Fluor und/ oder Chlor substituiert ist), $C_1$-$C_4$-Alkylthio, $C_1$-$C_4$-Alkylsulfinyl oder $C_1$-$C_4$-Alkylsulfonyl (welches gegebenenfalls durch Fluor und/oder Chlor substituiert ist), Di-($C_1$-$C_4$-alkyl)-amino-sulfonyl oder $C_1$-$C_4$-Alkoxy-carbonyl stehen, und

$A^1$

für Sauerstoff, Schwefel oder die Gruppierung N-$Z^1$ steht, wobei

$Z^1$

für Wasserstoff, $C_1$-$C_4$-Alkyl (welches gegebenenfalls durch Fluor, Chlor, Brom oder Cyano substituiert ist), $C_3$-$C_6$-Cycloalkyl, Benzyl, Phenyl (welches gegebenenfalls durch Fluor, Chlor, Brom oder Nitro substituiert ist), $C_1$-$C_4$-Alkylcarbonyl, $C_1$-$C_4$-Alkoxy-carbonyl oder Di-($C_1$-$C_4$-alkyl)-aminocarbonyl steht; worin weiter

R für den Rest

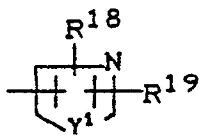

steht, worin

$R^{18}$ und $R^{19}$

gleich oder verschieden sind und für Wasserstoff, $C_1$-$C_4$-Alkyl, Halogen, $C_1$-$C_4$-Alkoxycarbonyl, $C_1$-$C_4$-Alkoxy oder $C_1$-$C_4$-Halogenalkoxy stehen,

$Y^1$

für Schwefel oder die Gruppierung N-$R^{20}$ steht, wobei

$R^{20}$

für Wasserstoff oder $C_1$-$C_4$-Alkyl steht; worin weiter

R für den Rest

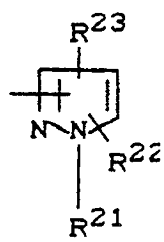

steht, worin

$R^{21}$

für Wasserstoff, $C_1$-$C_4$-Alkyl, Phenyl oder (Iso)Chinolinyl steht,

$R^{22}$

für Wasserstoff, Halogen, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), Dioxolanyl oder $C_1$-$C_4$-Alkoxy-carbonyl steht und

$R^{23}$

für Wasserstoff, Halogen oder $C_1$-$C_4$-Alkyl steht; worin weiter

R für den Rest

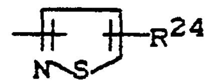

steht, worin

$R^{24}$

für Wasserstoff, Halogen, $C_1$-$C_4$-Alkyl oder $C_1$-$C_4$-Alkoxy-carbonyl steht; worin weiter

R für den Rest

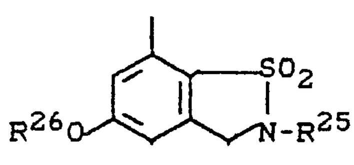

steht, worin

$R^{25}$

für $C_1$-$C_4$-Alkyl steht und

$R^{26}$

für $C_1$-$C_4$-Alkyl steht, worin weiter

R für den Rest

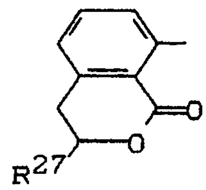

steht, worin

$R^{27}$

für Wasserstoff oder Methyl steht;

in welcher weiter

X für Wasserstoff, Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy oder $C_1$-$C_4$-Halogenalkoxy steht,

Y für Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy oder $C_1$-$C_4$-Halogenalkoxy steht und

Z für Wasserstoff, Halogen, $C_1$-$C_4$-Alkyl, $C_1$-$C_4$-Halogenalkyl, $C_1$-$C_4$-Alkoxy oder $C_1$-$C_4$-Halogenalkoxy steht.

Die Erfindung betrifft weiter vorzugsweise Salze von Verbindungen der Formel (I)

α) mit Protonensäuren, wie z. B. Salzsäure, Schwefelsäure, Phosphorsäure, Methansulfonsäure, Benzol- oder p-Toluolsulfonsäure, oder Naphthalin-mono- oder -di-sulfonsäuren, oder

β) mit Basen, wie z. B. Natrium-, Kalium- oder Calcium-hydroxid, -hydrid, -amid oder-carbonat, Natrium- oder Kalium-$C_1$-$C_4$-alkanolaten, Ammoniak, $C_1$-$C_4$-Alkylaminen, Di-($C_1$-$C_4$-alkyl)-aminen oder Tri-($C_1$-$C_4$-alkyl)-aminen.

Gegenstand der Erfindung sind insbesondere Verbindungen der Formel (I), in welcher

R für den Rest

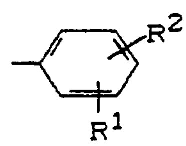

steht, worin

$R^1$

für Wasserstoff, Fluor, Chlor, Brom, Cyano, Nitro, Carboxy, Methyl, Trifluormethyl, Methoxy, Ethoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, Diethylaminosulfonyl, N-Methoxy-N-methylaminosulfonyl, Phenyl, Phenoxy oder $C_1$-$C_3$-Alkoxy-carbonyl steht und

$R^2$

für Wasserstoff, Fluor, Chlor, Brom, Methyl, Trifluormethyl oder Methoxy steht; worin weiter

R für den Rest

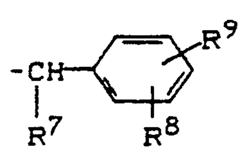

steht, worin

$R^7$

für Wasserstoff steht,

$R^8$

für Fluor, Chlor, Brom, Cyano, Carboxy, Methyl, Trifluormethyl, Methoxy, Difluormethoxy, Trifluormethoxy, Ethoxy, Methoxycarbonyl, Ethoxycarbonyl, Methylsulfonyl oder Dimethylaminosulfonyl

steht und
$R^9$
für Wasserstoff, Fluor oder Chlor steht; worin weiter
R für den Rest

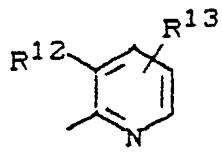

steht, worin
$R^{12}$
für Dimethylaminocarbonyl oder $C_1$-$C_4$-Alkylsulfonyl steht und
$R^{13}$
für Wasserstoff, Fluor oder Chlor steht;
worin weiter
R für den Rest

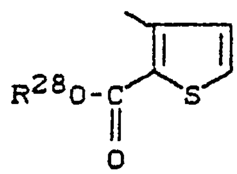

steht, worin
$R^{28}$
für Methyl oder Ethyl steht, oder
R für den Rest

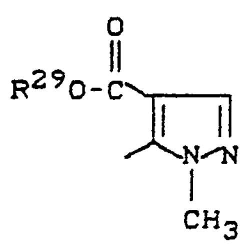

steht, worin
$R^{29}$
für Methyl oder Ethyl steht,
in welcher weiter
X für Wasserstoff, Fluor, Chlor, Brom, Methyl, Ethyl, Trifluormethyl, Methoxymethyl, Methoxy, Ethoxy oder Difluormethoxy steht,
Y für Fluor, Chlor, Brom, Methyl, Ethyl, Fluormethyl, Chlormethyl, Difluormethyl, Dichlormethyl, Trifluormethyl, Trichlormethyl, Chlordifluormethyl, Fluordichlormethyl, Methoxy, Ethoxy, Difluormethoxy oder Trifluormethoxy steht und
Z für Wasserstoff, Fluor, Chlor, Brom, Methyl, Ethyl, Trifluormethyl, Methoxy, Ethoxy oder Difluormethoxy steht.

Eine ganz besonders bevorzugte Gruppe von Verbindungen der Formel (I) sind diejenigen, bei welchen
R die oben als insbesondere bevorzugt angegebene Bedeutung hat,
X für Wasserstoff steht,
Y für Methoxy oder Methyl steht und
Z für Wasserstoff steht.

Verwendet man beim erfindungsgemäßen Verfahren (a) beispielsweise 2-Fluor-benzolsulfonsäurechlorid und 5-Methoxy-pyrimidin-2-carbonsäureamid als Ausgangsstoffe, so kann der Reaktionsablauf durch das folgende Formelschema skizziert werden:

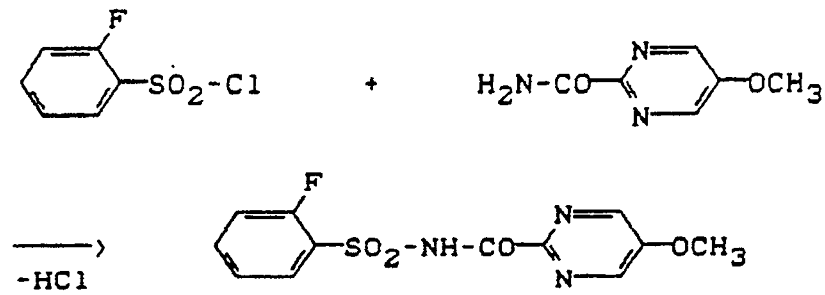


Verwendet man beim erfindungsgemäßen Verfahren (b) beispielsweise 2-Trifluormethoxy-benzylsulfonamid und 4,5,6-Trimethyl-pyrimidin-2-carbonsäuremethylester als Ausgangsstoffe, so kann der Reaktionsablauf durch das folgende Formelschema skizziert werden:

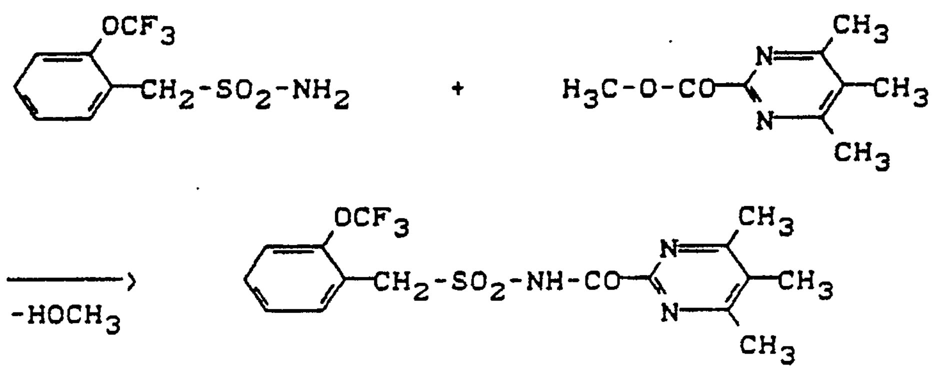


Die beim erfindungsgemäßen Verfahren (a) zur Herstellung von Verbindungen der Formel (I) als Ausgangsstoffe zu verwendenden Pyrimidincarbonsäureamide sind durch die Formel (II) allgemein definiert.

In Formel (II) haben X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für X, Y und Z angegeben wurden.

Als Beispiele für die Ausgangsstoffe der Formel (II) seien genannt:

5-Fluor-, 5-Chlor-, 5-Brom-, 5-Methyl-, 5-Ethyl-, 5-Methoxy- und 5-Ethoxy-pyrimidin-2-carbonsäureamid sowie 5-Fluor-4,6-dimethyl-, 5-Chlor-4,6-dimethyl-, 5-Brom-4,6-dimethyl- und 4,5,6-Trimethyl-pyrimidin-2-carbonsäureamid.

Die Ausgangsstoffe der Formel (II) sind bekannt und/oder können nach an sich bekannten Verfahren hergestellt werden (vgl. GB-PS 777465; Compt. Rend. 248 (1959), 250-252 - zitiert in Chem. Abstracts 53, 18049i; Ann. Chim. (Paris) 5 (1960), 351-379 - zitiert in Chem. Abstracts 56, 5961g; Collect. Czech. Chem. Commun. 37 (1972), 1721-1733 - zitiert in Chem. Abstracts 77, 101522s; Org. React. (Tartu) 20 (1983), 85-95 - zitiert in Chem. Abstracts 99, 157694w).

Die beim erfindungsgemäßen Verfahren (a) zur Herstellung der neuen Verbindungen der Formel (I) weiter als Ausgangsstoffe zu verwendenden Sulfonsäurehalogenide bzw. -anhydride sind durch die Formel (III) allgemein definiert. In Formel (III) hat R vorzugsweise bzw. insbesondere diejenige Bedeutung, die oben im Rahmen der Beschreibung der erfindungsgemäßen Verbindungen der Formel (I) vorzugsweise bzw. als insbesondere bevorzugt für R angegeben wurde und Q steht vorzugsweise für Chlor.

Als Ausgangsstoffe der Formel (III) seien beispielsweise genannt:

Benzolsulfonsäurechlorid, 2-Chlor-, 3-Chlor-, 4-Chlor-, 2,5-Dichlor-, 2-Fluor-, 4-Fluor-, 2-Brom-, 4-Brom-, 2-Cyano-, 2-Nitro-, 4-Nitro-, 2-Methyl-, 4-Methyl-, 2-Chlormethyl-, 2-Trifluormethyl-, 2-Methoxy-, 4-Methoxy-, 2-Methylthio-, 2-Trifluormethylthio-, 2-Difluormethylthio-, 2-Cyclopropyloxycarbonyl-, 2-Phenoxy-, 2-Difluormethoxy-, 2-Trifluormethoxy-, 2-(2-Chlorethoxy)-, 2-Methylthiomethyl-, 2-Dimethylaminosulfonyl-, 2-Phenyl-, 2-Methoxycarbonyl-, 2-Ethoxycarbonyl-, 2-Dimethylaminocarbonyl- und 2-Diethylaminocarbonyl-benzolsulfonsäurechlorid sowie (2-Chlor-phenyl)-, (2-Cyano-phenyl)-, (2-Methcxycarbonyl-phenyl)- und (2-

Trifluormethoxy-phenyl)-methansulfonsäurechlorid, 2-Chlor-6-methyl-benzolsulfonsäurechlorid und 2,6-Dichlor-benzolsulfonsäurechlorid.

Die Sulfonsäurehalogenide bzw. -anhydride der Formel (III) sind bekannt und/oder können nach an sich bekannten Verfahren hergestellt werden (vgl. J. Org. Chem. 33 (1968), 2104; J. Org. Chem. 25 (1960), 1824; DE-AS 2 308 262; EP-OS 23 140, 23 141, 23 422, 35 893, 48 143, 51 466, 64 322, 70 041, 44 808 und 44 809; US-PS 2 929 820, 4 282 242; 4 348 220 und 4 372 778 sowie Angew. Chem. 93 (1981), 151).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid und Pyridin.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z B. Calciumhydroxid, Alkalimetallhydride wie z.B. Natrium- und Kaliumhydrid, Erdalkalimetallhydride wie z.B. Calciumhydrid, Alkalicarbonate und -alkoholate wie Natrium- und Kaliumcarbonat, Natrium- und Kalium-tert.-butylat, ferner aliphatische, aromatische oder heterocyclische Amine, beispielsweise Triethylamin, Trimethylamin, Dimethylanilin, Dimethylbenzylamin, Pyridin, Picolin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen -20 °C und +100 °C, vorzugsweise bei Temperaturen zwischen 0 °C und +80 °C.

Zur Durchführung des erfindungsgemäßen Verfahrens (a) setzt man je Mol Pyrimidincarbonsäureamid der Formel (II) im allgemeinen zwischen 1 und 5 Mol, vorzugsweise zwischen 1 und 3 Mol, Sulfonsäurehalogenid bzw. Sulfonsäureanhydrid der Formel (III) ein.

Die Reaktionskomponenten können in beliebiger Reihenfolge zusammengegeben werden.
In einer bevorzugten Ausführungsform des erfindungsgemäßen Verfahrens (a) werden eine Ausgangsverbindung der Formel (II) und ein Säureakzeptor mit einem Verdünnungsmittel verrührt, und in diese Mischung wird eine Ausgangsverbindung der Formel (III) langsam (gegebenenfalls portionsweise) eindosiert. Das Reaktionsgemisch wird dann bis zum Ende der Umsetzung gerührt.

Die Aufarbeitung kann nach üblichen Methoden durchgeführt werden (vgl. die Herstellungsbeispiele).

Die beim erfindungsgemäßen Verfahren (b) zur Herstellung von Verbindungen der Formel (I) als Ausgangsstoffe zu verwendenden Pyrimidincarbonsäuren sind durch die Formel (IV) allgemein definiert.

In Formel (IV) haben X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für X, Y und Z angegeben wurden.

Als Beispiele für die Ausgangsstoffe der Formel (IV) seien genannt:
5-Fluor-, 5-Chlor-, 5-Brom-, 5-Methyl-, 5-Ethyl-, 5-Methoxy- und 5-Ethoxy-pyrimidin-2-carbonsäure sowie 5-Fluor-4,6-dimethyl-, 5-Chlor-4,6-dimethyl-, 5-Brom-4,6-dimethyl- und 4,5,6-Trimethyl-pyrimidin-2-carbonsäure.

Die Ausgangsstoffe der Formel (IV) sind bekannt und/oder können nach an sich bekannten Verfahren hergestellt werden (vgl. GB-PS 777465; Compt. Rend. 248 (1959), 250-252 - zitiert in Chem. Abstracts 53, 18049i; Ann. Chim. (Paris) 5 (1960), 351-379 - zitiert in Chem. Abstracts 56, 5961g; Collect. Czech. Chem. Commun. 37 (1972), 1721-1733 - zitiert in Chem. Abstracts 77, 101522s; Org. React. (Tartu) 20 (1983), 85-95 - zitiert in Chem. Abstracts 99, 157694w; Chem. Pharm. Bull. 28 (1980), 1408-1414 - zitiert in Chem. Abstracts 94, 47251p).

Anstatt der Pyrimidincarbonsäuren der Formel (IV) werden beim erfindungsgemäßen Verfahren (b) gegebenenfalls die davon abgeleiteten Carbonsäurechloride oder davon abgeleitete Alkylester (vorzugsweise Methylester oder Ethylester), Aralkylester (vorzugsweise Benzylester) oder Arylester (vorzugsweise Phenylester, in der Phenylgruppe gegebenenfalls durch Cyano, Nitro, Chlor, Fluor, Brom und/oder Methyl substituiert) als Ausgangsstoffe ("reaktionsfähige Derivate") eingesetzt.

Man erhält die von den Verbindungen der Formel (IV) abgeleiteten Carbonsäurechloride aus den entsprechenden Carbonsäuren nach üblichen Methoden, beispielsweise durch Umsetzung mit üblicherweise

als "Chlorierungsmittel" verwendeten Säurechloriden, wie z.B. Phosgen, Oxalylchlorid oder Thionylchlorid, gegebenenfalls in Gegenwart von Reaktionshilfsmitteln, wie z.B. Pyridin, und gegebenenfalls in Gegenwart von Verdünnungsmitteln, wie z.B. Chloroform oder Tetrachlormethan, bei Temperaturen zwischen 0 °C und 100 °C.

Die als weitere reaktionsfähige Derivate der Verbindungen der Formel (IV) genannten Ester können aus den entsprechenden Carbonsäurechloriden und geeigneten Alkoholen oder Phenolen nach üblichen Methoden, im allgemeinen durch Umsetzung in Gegenwart eines Säureakzeptors, wie z.B. Triethylamin oder Pyridin, und in Gegenwart eines Verdünnungsmittels, wie z.B. Acetonitril, bei Temperaturen zwischen 0 °C und 100 °C erhalten werden.

Man kann die genannten Ester jedoch auch direkt aus den Carbonsäuren der Formel (IV) in Gegenwart von Kondensationshilfsmitteln, wie z.B. Dicyclohexylcarbodiimid, gegebenenfalls in Gegenwart von Reaktionshilfsmitteln, wie z.B. 4-Dimethylamino-pyridin, und gegebenenfalls in Gegenwart von Verdünnungsmitteln, wie z.B. Methylenchlorid oder Chloroform, bei Temperaturen zwischen 0 °C und 100 °C erhalten.

Die beim erfindungsgemäßen Verfahren (b) zur Herstellung von Verbindungen der Formel (I) weiter als Ausgangsstoffe zu verwendenden Sulfonsäureamide sind durch die Formel (V) allgemein definiert.

In Formel (V) hat R vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R angegeben wurde.

Als Beispiele für die Ausgangsstoffe der Formel (V) seien genannt:

Benzolsulfonsäureamid, 2-Chlor-, 3-Chlor-, 4-Chlor-, 2,5-Dichlor-, 2-Fluor-, 4-Fluor-, 2-Brom-, 4-Brom-, 2-Cyano-, 2-Nitro-, 4-Nitro-, 2-Methyl-, 4-Methyl-, 2-Chlormethyl-, 2-Trifluormethyl-, 2-Methoxy-, 4-Methoxy-, 2-Methylthio-, 2-Trifluormethylthio-, 2-Difluormethylthio-, 2-Cyclopropyloxycarbonyl-, 2-Phenoxy-, 2-Difluormethoxy, 2-Trifluormethoxy-, 2-(2-Chlorethoxy)-, 2-Methylthiomethyl-, 2-Dimethylaminosulfonyl-, 2-Phenyl-, 2-Methoxycarbonyl-, 2-Ethoxycarbonyl-, 2-Dimethylaminocarbonyl- und 2-Diethylaminocarbonyl-benzolsulfonsäureamid sowie (2-Chlor-phenyl)-, (2-Cyano-phenyl)-, (2-Methoxycarbonyl-phenyl)- und (2-Trifluormethoxy-phenyl)-methansulfonsäureamid, 2-Chlor-6-methyl-benzolsulfonsäurechlorid und 2,6-Dichlorbenzolsulfonsäureamid.

Die Sulfonsäureamide der Formel (V) sind bekannt und/oder können nach an sich bekannten Verfahren hergestellt werden (vgl. J. Org. Chem. 33 (1968), 2104; J. Org. Chem. 25 (1960), 1824; DE-AS 2 308 262; EP-A 23 140, 23 141, 23 422, 35 893, 48 143, 51 466, 64 322, 70 041, 44 808 und 44 809; US-P 2 929 820, 4 282 242; 4 348 220 und 4 372 778 sowie Angew. Chem. 93 (1981), 151).

Anstatt der Sulfonsäureamide der Formel (V) werden beim erfindungsgemäßen Verfahren (b) gegebenenfalls die davon abgeleiteten Sulfonylisocyanate ("reaktionsfähige Derivate") als Ausgangsstoffe eingesetzt. Diese Verbindungen sind bekannt und/oder können nach an sich bekannten Verfahren hergestellt werden (vgl. J. Org. Chem. 31 (1966), 2658-2661; EP-A 7687; EP-A 46626; EP-A 21641; EP-A 23140; EP-A 23141; EP-A 70041; EP-A 23422, EP-A 64322; EP-A 34431; EP-A 35893; EP-A 51466, EP-A 44808; EP-A 173312; DE-OS 3132944; EP-A 87780; EP-A 271780).

Das erfindungsgemäße Verfahren (b) zur Herstellung der neuen Verbindungen der Formel (I) wird vorzugsweise unter Verwendung von Verdünnungsmitteln durchgeführt. Es kommen hierbei vorzugsweise die gleichen Verdünnungsmittel in Betracht, die oben für das erfindungsgemäße Verfahren (a) angegeben sind.

Das erfindungsgemäße Verfahren (b) wird gegebenenfalls in Gegenwart eines Reaktionshilfsmittels durchgeführt. Geeignete Reaktionshilfsmittel sind hierbei Verbindungen, mit denen Carbonsäuren in reaktionsfähige Intermediate überführt werden, die dann in situ mit nucleophilen Verbindungen, wie beispielsweise den Sulfonsäureamiden der Formel (V) zu entsprechenden Carbonsäurederivaten umgesetzt werden. Als Beispiele für derartige Reaktionshilfsmittel seien Carbonyldiimidazol und 2-Chlor-1-methyl-pyridiniumiodid genannt.

Für die Umsetzung der als reaktionsfähige Derivate von Verbindungen der Formel (IV) genannten Carbonsäurechloride und Ester mit Sulfonsäureamiden der Formel (V) sind auch Säureakzeptoren, wie sie oben für das erfindungsgemäße Verfahren (a) angegeben sind, als Reaktionshilfsmittel geeignet.

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im

allgemeinen in einem geeigneten Verdünnungsmittel gegebenenfalls in Gegenwart eines Reaktionshilfsmittels durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z. B. Wasser, Methanol, Ethanol, Methylenchlorid oder Aceton, und Zugabe einer geeigneten Säure bzw. Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen eignen sich sehr gut zur selektiven Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate

aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methyl-thio-1,2,4-triazin-5(4H)-on (METRIBUZIN) zur Unkrautbekämpfung in Sojabohnen, infrage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitro-benzoesäure (ACIFLUORFEN); Chloressigsäure-N-(methoxy-methyl)2,6-diethylanilid (ALACHLOR); Methyl-6,6-dimethyl-2,4-dioxo-3-[1-(2-propenyloxyamino)-butyliden]-cyclohexancarbonsäure (ALLOXYDIM); 4-Amino-benzolsulfonyl-methylcarbamat (ASULAM); 2-Chlor-4-ethylamino-6-isopropylamino-1,3,5-triazin (ATRAZIN); 2-[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid (BUTACHLOR); 5-Amino-4-chlor-2-phenyl-2,3-dihydro-3-oxypyridazin (CHLORIDAZON); Ethyl-2-{[(4-chlor-6-methoxy-2-pyrimidinyl)-aminocarbonyl]-aminosulfonyl}-benzoat (CHLORIMURON); N-(3-Chlorphenyl)-isopropylcarbamat (CHLORPROPHAM); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); exo-1-Methyl-4-(1-methyl-ethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); N,S-Diethyl-N-cyclohexylthiolcarbamat (CYCLOATE); 2-[1-(Ethoximino)-butyl]-3-hydroxy-5-[tetrahydro-(2H)-thiopyran-3-yl]-2-cyclohexen-1-on (CYCLOXYDIM); 2-[4-(2,4-Dichlorphenoxy)-phenoxy]-propionsäure, deren Methyl- oder deren Ethylester (DICLOFOP); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor2-benzoxazolyl)-oxy]-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); [(4-Amino-3,5-dichlor-6-fluor-2-pyridinyl)-oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincar bonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-N'-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[-(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); S-Ethyl-N,N-hexamethylen-thiolcarbamat (MOLINATE); 1-(3-Trifluormethyl-phenyl)-4-methylamino-5-chlor-6-pyridazon (NORFLURAZON); 4-(Di-n-propylamino)-3,5-dinitrobenzolsulfonamid (ORYZALIN); (2-Chlor-4-trifluormethylphenyl)-(3-ethoxy-4-nitrophenyl)-ether (OXYFLUORFEN); N-(1-Ethylpropyl)-3,4-dimethyl-2,6-dinitroanilin (PENDIMETHALIN); 3-(Ethoxycarbonylaminophenyl)-N-(3'-methylphenyl)-carbamat (PHENMEDIPHAM); 2-Chlor-N-isopropylacetanilid (PROPACHLOR); Isopropyl-N-phenyl-carbamat (PROPHAM); 0-(6-Chlor-3-phenyl-pyridazin-4-yl)-S-octyl-thiocarbamat (PYRIDATE); 2-[4-(6-Chlor-chinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); S-[(4-Chlorphenyl)-methyl]-N,N-diethyl-thiocarbamat (THIOBENCARB); N,N-Diisopropyl-S-(2,3,3-trichlorallyl)-thiolcarbamat (TRIALLATE); 2-6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,1 g und 2000 g Wirkstoff pro Hektar Bodenfläche, vorzugsweise zwischen 1 g und 1000 g pro ha.

Die Herstellung und die Verwendung der erfindungsgemäßen Wirkstoffe geht aus den nachfolgenden Beispielen hervor.

Herstellungsbeispiele:

Beispiel 1

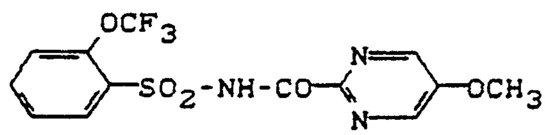

(Verfahren (a))

Eine Mischung aus 3,1 g (0,02 Mol) 5-Methoxy-pyrimidin-2-carbonsäureamid, 3,4 g (0,06 Mol) Kaliumhydroxid (Pulver) und 50 ml Dioxan wird auf 80° C erhitzt und 10 Minuten bei dieser Temperatur gerührt. Nach Abkühlen auf 20° C werden 10,4 g (0,04 Mol) 2-Trifluormethoxy-benzolsulfonsäurechlorid dazugegeben und das Reaktionsgemisch wird 20 Stunden bei 20° C gerührt. Dann wird eingeengt, der Rückstand mit 100 ml 10%iger Kaliumcarbonat-Lösung verrührt, filtriert und das Filtrat mit 2N-Salzsäure angesäuert. Das ölig abgeschiedene Produkt wird in Methylenchlorid aufgenommen, diese Lösung wird mit Natriumsulfat getrocknet und filtriert. Das Filtrat wird eingeengt, der Rückstand mit Diethylether verrieben und das hierbei kristallin angefallene Produkt durch Absaugen isoliert.

Man erhält 0,7 g (9% der Theorie) N-(2-Trifluormethoxy-phenylsulfonyl)-5-methoxy-pyrimidin-2-carbonsäureamid vom Schmelzpunkt 187° C.

Beispiel 2

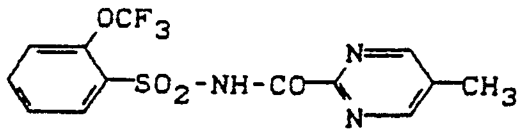

(Verfahren (b))

Eine Mischung aus 1,46 g (6,8 mmol) 5-Methyl-pyrimidin-2-carbonsäure-phenylester, 1,64 g (6,8 mmol) 2-Trifluormethoxy-benzolsulfonamid, 1,04 g (6,8 mmol) 1,8-Diazabicyclo-[5,4,0]-undec-7-en (DBU) und 40 ml Acetonitril wird 7 Stunden bei 70° C gerührt und anschließend eingeengt. Der Rückstand wird mit 2N-Salzsäure verrührt und das ungelöst gebliebene Produkt durch Absaugen isoliert.

Man erhält 1,74 g (71% der Theorie) N-(2-Trifluormethoxy-phenylsulfonyl)-5-methyl-pyrimidin-2-carbonsäureamid vom Schmelzpunkt 123° C.

Analog zu den Beispielen 1 und 2 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

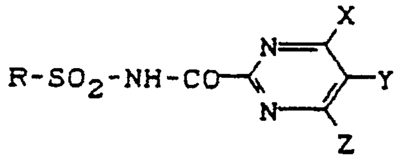

(I)

Tabelle 1: Beispiele für die Verbindungen der Formel (I)

| Bsp.-Nr. | R | X | Y | Z | Schmelzpunkt (°C) |
|---|---|---|---|---|---|
| 3 | 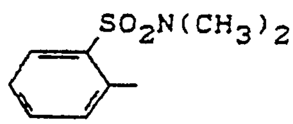 | H | $OCH_3$ | H | 226 |
| 4 | 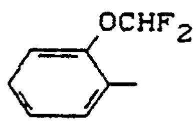 | H | $CH_3$ | H | 141 |
| 5 | 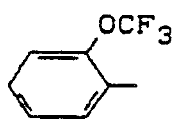 | $CH_3$ | $CH_3$ | $CH_3$ | 114 |
| 6 | 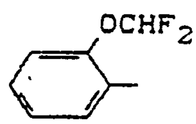 | $CH_3$ | $CH_3$ | $CH_3$ | 130 |
| 7 | 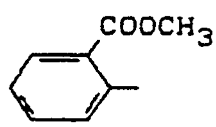 | H | $OCH_3$ | H | 106 |

Beispiel zur Herstellung von Ausgangsstoffen:

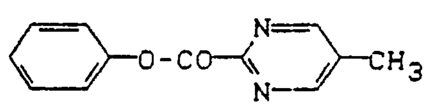

4 g (0,029 Mol) 5-Methyl-pyrimidin-2-carbonsäure und 3 g (0,032 Mol) Phenol werden in 50 ml Methylenchlorid vorgelegt und nacheinander werden 0,36 g (0,003 Mol) 4-Dimethylamino-pyridin und 6,67 g (0,032 Mol) N,N'-Dicyclohexylcarbodiimid zugegeben. Man rührt das Reaktionsgemisch 12 Stunden bei Raumtemperatur und filtriert. Das Filtrat wird mit 5%iger Essigsäure und Wasser gewaschen, die organische Phase wird abgetrennt, über Natriumsulfat getrocknet, filtriert und eingeengt.

Es verbleiben 3,5 g (56% der Theorie) 5-Methylpyrimidin-2-carbonsäurephenylester als Feststoff vom

Schmelzpunkt 115° C.

Anwendungsbeispiele:

In den Anwendungsbeispielen werden die nachstehend aufgeführten Verbindungen (A) und (B) als Vergleichssubstanzen herangezogen:

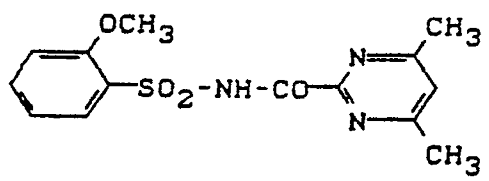

(A)

N-(2-Methoxy-phenylsulfonyl)-4,6-dimethyl-pyrimidin-2-carbonsäureamid
(bekannt aus EP-A 353640)

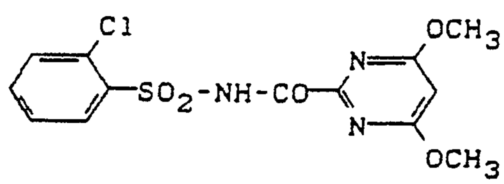

(B)

N-(2-Chlor-phenylsulfonyl)-4,6-dimethoxy-pyrimidin-2-carbonsäureamid
(bekannt aus EP-A 353640).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden, Die Konzentration der Spritzbrühe wird so gewählt, daß in 1000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß den Herstellungsbeispielen 1 und 3.

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen, Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigt in diesem Test z.B. die Verbindung gemäß Herstellungsbeispiel 1.

## Patentansprüche

1. Sulfonylierte Pyrimidincarbonsäureamide der allgemeinen Formel (I),

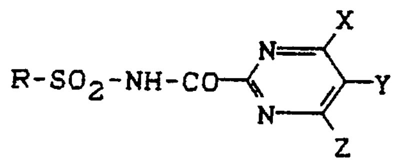

(I)

in welcher

R für jeweils gegebenenfalls substituiertes Aryl, Aralkyl oder Heteroaryl steht,
X für Wasserstoff, Halogen, Alkyl, Halogenalkyl, Alkoxyalkyl, Alkoxy oder Halogenalkoxy steht,
Y für Halogen, Alkyl, Halogenalkyl, Alkoxyalkyl, Alkoxy oder Halogenalkoxy steht und
Z für Wasserstoff, Halogen, Alkyl, Halogenalkyl, Alkoxy oder Halogenalkoxy steht,

sowie Salze von Verbindungen der Formel (I).

2. Sulfonylierte Pyrimidincarbonsäureamide der allgemeinen Formel (I) gemäß Anspruch 1, dadurch gekennzeichnet, daß darin

R für den Rest

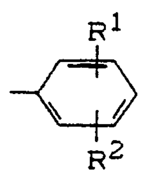

steht, worin

$R^1$ und $R^2$

gleich oder verschieden sind und für Wasserstoff, Fluor, Chlor, Brom, Iod, Cyano, Nitro, Carboxy, $C_1$-$C_6$-Alkyl (welches gegebenenfalls durch Fluor, Chlor, Brom, Cyano, Carboxy, $C_1$-$C_4$-Alkoxycarbonyl, $C_1$-$C_4$-Alkyl-amino-carbonyl, Di-($C_1$-$C_4$-alkyl)-amino-carbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-amino-sulfonyl, $C_3$-$C_6$-Cycloalkyl oder Phenyl substituiert ist), für $C_2$-$C_6$-Alkenyl (welches gegebenenfalls durch Fluor, Chlor, Brom, Cyano, $C_1$-$C_4$-Alkoxy-carbonyl, Carboxy oder Phenyl substituiert ist), für $C_2$-$C_6$-Alkinyl (welches gegebenenfalls durch Fluor, Chlor, Brom, Cyano, $C_1$-$C_4$-Alkoxy-carbonyl, Carboxy oder Phenyl substituiert ist), für $C_1$-$C_4$-Alkoxy (welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist), für $C_1$-$C_4$-Alkylthio (welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist), für $C_3$-$C_6$-Alkenyloxy (welches gegebenenfalls durch Fluor, Chlor, Brom, Cyano oder $C_1$-$C_4$-Alkoxy-carbonyl substituiert ist), für $C_2$-$C_6$-Alkenylthio (welches gegebenenfalls durch Fluor, Chlor, Brom, Cyano, Nitro, $C_1$-$C_3$-Alkylthio oder $C_1$-$C_4$-Alkoxy-carbonyl substituiert ist), $C_3$-$C_6$-Alkinyloxy, $C_3$-$C_6$-Alkinylthio oder für den Rest -$S(O)_p$-$R^3$ stehen, wobei

p

für die Zahlen 1 oder 2 steht und

$R^3$

für $C_1$-$C_4$-Alkyl (welches gegebenenfalls durch Fluor, Chlor, Brom, Cyano oder $C_1$-$C_4$-Alkoxy-carbonyl substituiert ist), $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino oder für den Rest -NHOR$^4$ steht, wobei

$R^4$

für $C_1$-$C_6$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl )-aminocarbonyl substituiert ist), für $C_3$-$C_6$-Alkenyl (welches gegebenenfalls durch Fluor, Chlor oder Brom substituiert ist), $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist), für Benzhydryl oder für Phenyl (welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist) steht,

$R^1$ und $R^2$

weiterhin für Phenyl oder Phenoxy, für $C_1$-$C_4$-Alkylcarbonylamino, $C_1$-$C_4$-Alkoxy-carbonylamino, $C_1$-$C_4$-Alkyl-amino-carbonyl-amino, Di-($C_1$-$C_4$-alkyl)-amino-carbonylamino, oder für den Rest -CO-R$^5$ stehen, wobei

$R^5$

für $C_1$-$C_6$-Alkyl, $C_1$-$C_6$-Alkoxy (welches gegebenenfalls durch Fluor, Chlor, Methoxy oder Ethoxy substituiert ist), $C_3$-$C_6$-Cycloalkoxy, $C_3$-$C_6$-Alkenyloxy, $C_1$-$C_4$-Alkylthio, Amino, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht (welche gegebenenfalls durch Fluor und/oder Chlor substituiert sind),

$R^1$ und $R^2$

weiterhin für $C_1$-$C_4$-Alkylsulfonyl-oxy, Di-($C_1$-$C_4$-alkyl)-aminosulfonyl-amino oder für den Rest -CH = N-R$^6$ stehen, wobei

$R^6$

für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/oder Chlor substituiertes $C_1$-$C_6$-Alkoxy, $C_3$-$C_6$-Alkenoxy, $C_3$-$C_6$-Alkinoxy oder Benzyloxy, für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkyl-sulfonylamino oder für gegebenenfalls durch Fluor, Chlor, Brom oder Methyl substituiertes Phenylsulfonylamino steht,

worin weiter

R für den Rest

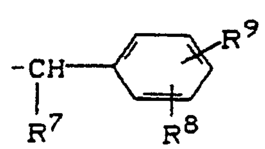

steht, worin

$R^7$

für Wasserstoff oder $C_1$-$C_4$-Alkyl steht,

$R^8$ und $R^9$

gleich oder verschieden sind und für Wasserstoff, Fluor, Chlor, Brom, Nitro, Cyano, Carboxy, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), $C_1$-$C_4$-Alkoxy-carbonyl, $C_1$-$C_4$-Alkylsulfonyl oder Di-($C_1$-$C_4$-alkyl)-aminosulfonyl stehen; worin weiter

R für den Rest

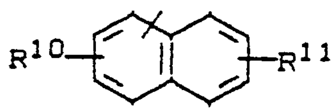

steht,
worin
$R^{10}$ und $R^{11}$
gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist) oder $C_1$-$C_4$-Alkoxy (welches gegebenenfalls durch Fluor und/oder Chlor substituiert ist), stehen; worin weiter

R für den Rest

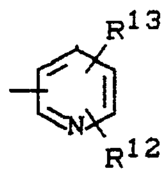

steht, worin
$R^{12}$ und $R^{13}$
gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_2$-$C_4$-Alkenyl (welches gegebenenfalls durch Fluor und/oder Chlor substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), sowie für Di-($C_1$-$C_4$-alkyl)-amino-sulfonyl, $C_1$-$C_4$-Alkoxy-carbonyl, Dimethylaminocarbonyl oder Dioxolanyl stehen; worin weiter

R für den Rest

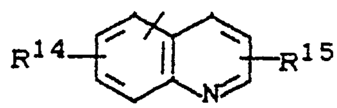

steht,
worin
$R^{14}$ und $R^{15}$
gleich oder verschieden sind und für Wasserstoff, Fluor, Chlor, Brom, $C_1$-$C_4$-Alkyl (welches gegebenenfalls durch Fluor und/oder Brom substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), oder für Di-($C_1$-$C_4$-alkyl)-amino-sulfonyl stehen; worin weiter

R für den Rest

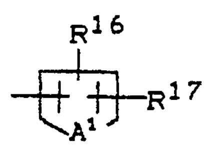

steht, worin
$R^{16}$ und $R^{17}$
gleich oder verschieden sind und für Wasserstoff, Fluor, Chlor, Brom, Cyano, Nitro, $C_1$-$C_4$-Alkyl (welches gegebenenfalls durch Fluor, Chlor, $C_1$-$C_4$-Alkoxy und/oder $C_1$-$C_4$-Halogenalkoxy substituiert ist), $C_1$-$C_4$-Alkoxy (welches gegebenenfalls durch Fluor und/ oder Chlor substituiert ist), $C_1$-$C_4$-Alkylthio, $C_1$-$C_4$-Alkylsulfinyl oder $C_1$-$C_4$-Alkylsulfonyl (welches gegebe-

nenfalls durch Fluor und/oder Chlor substituiert ist), Di-($C_1$-$C_4$-alkyl)-amino-sulfonyl oder $C_1$-$C_4$-Alkoxy-carbonyl stehen, und

$A^1$

für Sauerstoff, Schwefel oder die Gruppierung N-$Z^1$ steht, wobei

$Z^1$

für Wasserstoff, $C_1$-$C_4$-Alkyl (welches gegebenenfalls durch Fluor, Chlor, Brom oder Cyano substituiert ist), $C_3$-$C_6$-Cycloalkyl, Benzyl, Phenyl (welches gegebenenfalls durch Fluor, Chlor, Brom oder Nitro substituiert ist), $C_1$-$C_4$-Alkylcarbonyl, $C_1$-$C_4$-Alkoxy-carbonyl oder Di-($C_1$-$C_4$-alkyl)aminocarbonyl steht; worin weiter

R für den Rest

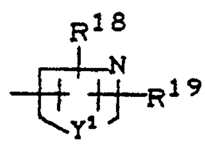

steht, worin

$R^{18}$ und $R^{19}$

gleich oder verschieden sind und für Wasserstoff, $C_1$-$C_4$-Alkyl, Halogen, $C_1$-$C_4$-Alkoxycarbonyl, $C_1$-$C_4$-Alkoxy oder $C_1$-$C_4$-Halogenalkoxy stehen,

$Y^1$

für Schwefel oder die Gruppierung N-$R^{20}$ steht, wobei

$R^{20}$

für Wasserstoff oder $C_1$-$C_4$-Alkyl steht; worin weiter

R für den Rest

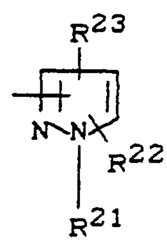

steht, worin

$R^{21}$

für Wasserstoff, $C_1$-$C_4$-Alkyl, Phenyl oder (Iso)Chinolinyl steht,

$R^{22}$

für Wasserstoff, Halogen, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), Dioxolanyl oder $C_1$-$C_4$-Alkoxy-carbonyl steht und

$R^{23}$

für Wasserstoff, Halogen oder $C_1$-$C_4$-Alkyl steht; worin weiter

R für den Rest

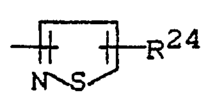

steht, worin

$R^{24}$

für Wasserstoff, Halogen, $C_1$-$C_4$-Alkyl oder $C_1$-$C_4$-Alkoxy-carbonyl steht; worin weiter

R für den Rest

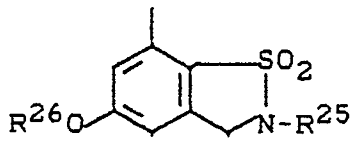

steht, worin
$R^{25}$
für $C_1$-$C_4$-Alkyl steht und
$R^{26}$
für $C_1$-$C_4$-Alkyl steht, worin weiter
R für den Rest

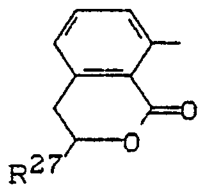

steht, worin
$R^{27}$
für Wasserstoff oder Methyl steht;
in welcher weiter
X für Wasserstoff, Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy oder $C_1$-$C_4$-Halogenalkoxy steht,
Y für Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy oder $C_1$-$C_4$-Halogenalkoxy steht und
Z für Wasserstoff, Halogen, $C_1$-$C_4$-Alkyl, $c_1$-$c_4$-Halogenalkyl, $C_1$-$C_4$-Alkoxy oder $C_1$-$C_4$-Halogenalkoxy steht.

3. Sulfonylierte Pyrimidincarbonsäureamide der allgemeinen Formel (1) gemäß Anspruch 1, dadurch gekennzeichnet, daß darin
R für den Rest

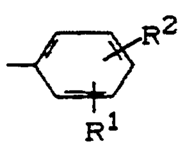

steht, worin
$R^1$
für Wasserstoff, Fluor, Chlor, Brom, Cyano, Nitro, Carboxy, Methyl, Trifluormethyl, Methoxy, Ethoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, Diethylaminosulfonyl, N-Methoxy-N-methylaminosulfonyl, Phenyl, Phenoxy oder $C_1$-$C_3$-Alkoxy-carbonyl steht und
$R^2$
für Wasserstoff, Fluor, Chlor, Brom, Methyl, Trifluormethyl oder Methoxy steht; worin weiter
R für den Rest

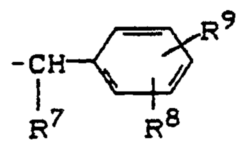

steht, worin
$R^7$
für Wasserstoff steht,
$R^8$
für Fluor, Chlor, Brom, Cyano, Carboxy, Methyl, Trifluormethyl, Methoxy, Difluormethoxy, Trifluormethoxy, Ethoxy, Methoxycarbonyl, Ethoxycarbonyl, Methylsulfonyl oder Dimethylaminosulfonyl steht und
$R^9$
für Wasserstoff, Fluor oder Chlor steht; worin weiter

R für den Rest

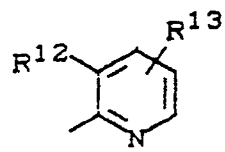

steht, worin
$R^{12}$
für Dimethylaminocarbonyl oder $C_1$-$C_4$-Alkylsulfonyl steht und
$R^{13}$
für Wasserstoff, Fluor oder Chlor steht;
worin weiter

R für den Rest

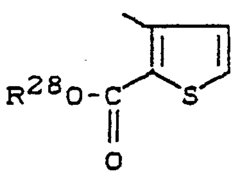

steht, worin
$R^{28}$
für Methyl oder Ethyl steht, oder

R für den Rest

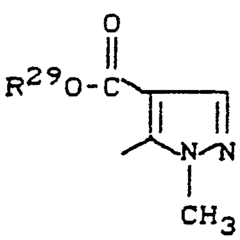

steht, worin
$R^{29}$
für Methyl oder Ethyl steht,

in welcher weiter

X für Wasserstoff, Fluor, Chlor, Brom, Methyl, Ethyl, Trifluormethyl, Methoxymethyl, Methoxy, Ethoxy oder Difluormethoxy steht,

Y für Fluor, Chlor, Brom, Methyl, Ethyl, Fluormethyl, Chlormethyl, Difluormethyl, Dichlormethyl, Trifluormethyl, Trichlormethyl, Chlordifluormethyl, Fluordichlormethyl, Methoxy, Ethoxy, Difluormethoxy oder Trifluormethoxy steht und

Z für Wasserstoff, Fluor, Chlor, Brom, Methyl, Ethyl, Trifluormethyl, Methoxy, Ethoxy oder Difluormethoxy steht.

4. Sulfonylierte Pyrimidincarbonsäureamide der allgemeinen Formel (I) gemäß Anspruch 1, dadurch gekennzeichnet, daß darin
   - R die in Anspruch 3 angegebene Bedeutung hat,
   - X für Wasserstoff steht,
   - Y für Methoxy oder Methyl steht und
   - Z für Wasserstoff steht.

5. Verfahren zur Herstellung von sulfonylierten Pyrimidincarbonsäureamiden der Formel (I),

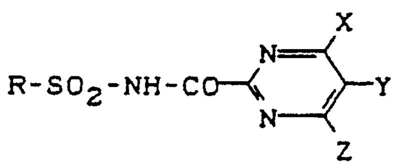
(I)

in welcher
   - R für jeweils gegebenenfalls substituiertes Aryl, Aralkyl oder Heteroaryl steht,
   - X für Wasserstoff, Halogen, Alkyl, Halogenalkyl, Alkoxyalkyl, Alkoxy oder Halogenalkoxy steht,
   - Y für Halogen, Alkyl, Halogenalkyl, Alkoxyalkyl, Alkoxy oder Halogenalkoxy steht und
   - Z für Wasserstoff, Halogen, Alkyl, Halogenalkyl, Alkoxy oder Halogenalkoxy steht,

sowie von Salzen von Verbindungen der Formel (I),
dadurch gekennzeichnet, daß man
(a) Pyrimidincarbonsäureamide der allgemeinen Formel (II)

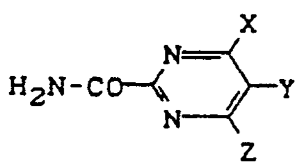
(II)

in welcher

X, Y und Z die oben angegebenen Bedeutungen haben,

mit Sulfonsäurehalogeniden oder Sulfonsäureanhydriden der allgemeinen Formel (III)

$R-SO_2-Q$ (III)

in welcher
   - R die oben angegebene Bedeutung hat und
   - Q für Fluor, Chlor, Brom oder die Gruppierung $-O-SO_2-R$ steht, worin R die oben angegebene Bedeutung hat, gegebenenfalls in Gegenwart eines Säureakzeptors und gegebenenfalls in Gegenwart eines Verdünnungsmittels umsetzt, oder daß man

(b) Pyrimidincarbonsäuren der allgemeinen Formel (IV)

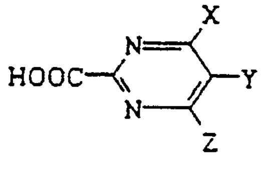
(IV)

in welcher

X, Y und Z die oben angegebenen Bedeutungen haben,

oder reaktionsfähige Derivate von Verbindungen der Formel (IV)

mit Sulfonsäureamiden der allgemeinen Formel (V)

$R-SO_2-NH_2$ (V)

in welcher
R die oben angegebene Bedeutung hat,
oder mit reaktionsfähigen Derivaten von Verbindungen der Formel (V)
gegebenenfalls in Gegenwart von Reaktionshilfsmitteln und gegebenenfalls in Gegenwart von Verdünnungsmitteln umsetzt
und gegebenenfalls die nach den Verfahren (a) oder (b) erhaltenen Produkte nach üblichen Methoden in Salze überführt.

**6.** Herbizide Mittel, gekennzeichnet durch einen Gehalt an mindestens einer Verbindung der Formel (I) gemäß Anspruch 1.

**7.** Verfahren zur Bekämpfung von Unkraut, dadurch gekennzeichnet, daß man eine Verbindung der Formel (I) gemäß Anspruch 1 auf die Unkräuter oder ihren Lebensraum einwirken läßt.

**8.** Verwendung von Verbindungen der Formel (I) gemäß Anspruch 1 zur Bekämpfung von unerwünschtem Pflanzenwachstum.

**9.** Verfahren zur Herstellung von herbiziden Mitteln, dadurch gekennzeichnet, daß man Verbindungen der Formel (I) gemäß Anspruch 1 mit Streckmitteln und/oder oberflächenaktiven Mitteln vermischt.

Europäisches Patentamt

# EUROPÄISCHER RECHERCHENBERICHT

Nummer der Anmeldung

EP 90124680.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| A | EP - A1 - 0 336 354 (HOECHST) * Ansprüche 1,3-7; Seiten 66-69 * -- | 1,5-9 | C 07 D 239/28<br>C 07 D 239/34<br>C 07 D 401/12<br>C 07 D 403/12<br>C 07 D 409/12<br>C 07 D 417/12<br>A 01 N 43/54 |
| D,A | EP - A2 - 0 353 640 (HOECHST) * Ansprüche 1,3,8-10; Seiten 144-150 * ---- | 1,5-9 | |
| | | | RECHERCHIERTE SACHGEBIETE (Int Cl 5) |
| | | | A 01 N 43/00<br>C 07 D 239/00<br>C 07 D 401/00<br>C 07 D 403/00<br>C 07 D 409/00<br>C 07 D 417/00 |

Der vorliegende Recherchenbericht wurde für alle Patentansprüche erstellt.

| Recherchenort | Abschlußdatum der Recherche | Prüfer |
|---|---|---|
| WIEN | 20-02-1991 | LUX |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82